(19) Europäisches Patentamt
European Patent Office
Office européen des brevets

(11) Veröffentlichungsnummer: **0 132 839**
**B1**

(12) # EUROPÄISCHE PATENTSCHRIFT

(45) Veröffentlichungstag der Patentschrift: **04.02.87**

(51) Int. Cl.⁴: **C 07 C 43/18**, C 07 C 41/06

(21) Anmeldenummer: **84108803.2**

(22) Anmeldetag: **25.07.84**

(54) **Neue Terpenether und Verfahren zu ihrer Herstellung.**

(30) Priorität: **27.07.83 DE 3327014**

(43) Veröffentlichungstag der Anmeldung: **13.02.85 Patentblatt 85/7**

(45) Bekanntmachung des Hinweises auf die Patenterteilung: **04.02.87 Patentblatt 87/6**

(84) Benannte Vertragsstaaten: **CH DE FR GB LI NL SE**

(56) Entgegenhaltungen:
**DE-A-2 815 392**
**DE-A-2 815 393**
**US-A-3 354 225**
**US-A-3 636 927**

(73) Patentinhaber: **HOECHST AKTIENGESELLSCHAFT, Postfach 80 03 20, D-6230 Frankfurt am Main 80 (DE)**

(72) Erfinder: **Scheidl, Franz, Dr., Goethestrasse 2, D-8901 Gablingen (DE)**
Erfinder: **Gscheidmeier, Manfred, Dr., Goethestrasse 4, D-8901 Gablingen (DE)**

EP 0 132 839 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Terpenether haben u.a. als Pflanzenschutzmittel (vgl. US-Patentschrift 38 71 863) und insbesondere als Riechstoffe (vgl. japanische Patentschrift 0 142 550; US-Patentschriften 36 36 927 und 33 54 225) wirtschaftliche Bedeutung. Es handelt sich dabei um Verbindungen der allgemeinen Formel

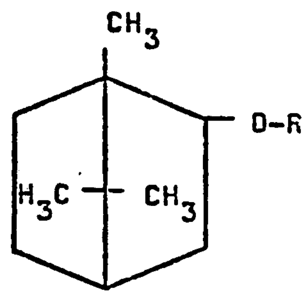

in der R für den Rest eines einfachen, kurzkettigen Mono- oder Dialkohols oder eines Derivats eines solchen steht.

Es wurde gefunden, daß bisher nicht bekennte Terpenether derselben allgemeinen Struktur, in denen jedoch R der Rest eines komplizierten, sterisch anspruchsvollen Alkohols ist, wertvolle Produkte bzw. Zwischenprodukte im Rahmen der vorstehend erwähnten Einsatzgebiete sowie auf dem Sektor der Fixateure und der Lösemittel darstellen.

Die Erfindung betrifft daher neue Terpenether der Formel

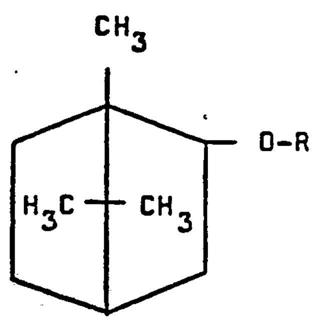

in welcher R die Bedeutung eines der nachstehend genannten Reste hat:

a) Cycloalkyl mit 5 bis 12 C-Atomen, welches durch einen Rest -O-$R^1$ substituiert sein kann, in dem R fur H, $C_1$ - bis $C_{18}$-Alkyl, $C_5$ - bis $C_{12}$ -Cycloalkyl, $C_7$ - bis $C_{12}$ -Bicycloalkyl, oder für cycloalkyl-, bicycloalkyl- oder tricycloalkylsubstituiertes $C_1$ - bis $C_6$ -Alkyl steht,

b) Bicycloalkyl mit 7 bis 12 C-Atomen,

c) $C_2$ - bis $C_{20}$ -Alkyl, welches durch einen Rest -O-$R^2$ substituiert ist, in dem $R^2$ die Bedeutung von $C_5$ - bis $C_{12}$ -Cycloalkyl, von $C_7$ - bis $C_{12}$ -Bicycloalkyl oder von cycloalkyl-, bicycloalkyl- oder tricycloalkylsubstituiertem $C_1$ - bis $C_6$-Alkyl hat,

d) $C_1$ - bis $C_4$ -Alkyl, das durch $C_5$ - bis $C_{12}$ -Cycloalkyl substituiert ist, wobei der Cycloalkylrest einen Substituenten -$CH_2$-O-$R^1$ mit $R^1$ wie unter a) angegeben tragen kann,

e) $C_1$ - bis $C_4$ -Alkyl, das durch $C_7$ - bis $C_{12}$ -Bicycloalkyl substituiert ist,

f) $C_1$ - bis $C_4$ -Alkyl, das durch $C_8$ - bis $C_{11}$ -Tricycloalkyl substituiert ist, wobei der Tricycloalkylrest seinerseits durch -$CH_2$-O-$R^1$ mit $R^1$ wie unter a angegeben substituiert sein kann, sowie ein Verfahren zu ihrer Synthese.

Bei den Terpenethern gemäß der Erfindung - nach IUPAC-Nomenklatur 1,7,7,Trimethyl-bicyclo-[2.2.1]-hept-2-yl-ethern - kann der 1,7,7-Trimethyl-bicyclo-[2.2.1]-hept-2-yl-Rest in der d-, loder vorzugsweise d,l-Form und der -O-R-Rest in der Exo- und/oder Endoform vorliegen.

Die neuen Verbindungen sind nach an sich bekannten Methoden herstellbar. So können sie beispielsweise nach dem klassischen Etherverfahren durch Umsetzen von Bornyl- und/oder Isobornylchlorid mit den Alkoholen, oder von Borneol und/oder Isoborneol mit den den Alkoholen entsprechenden Halogeniden der Formel R - X (X = Cl, Br, J) erhalten werden. Allerdings weist dieses Verfahren Nachteile auf, die vor allem in dem ökologisch unerwünschten Zwangsanfall von Halogenider, sowie der Bildung von Nebenprodukten

bestehen. Darüber hinaus ist, wenn vom Borneol oder Isoborneol ausgegangen werden soll, die Beschaffung der Halogenide - insbesondere der sich von komplizierten Alkoholen ableitenden - schwierig.

Die bessere und damit bevorzugte Verfahrensweise zur Herstellung der neuen Ether geht von dem wohlfeilen Camphen aus, das mit den Alkoholen der ellgemeinen Formel R-OH, wobei R die obengenannte Bedeutung hat, umgesetzt wird. Bei der in Gegenwart saurer Katalysatoren gemäß der Gleichung

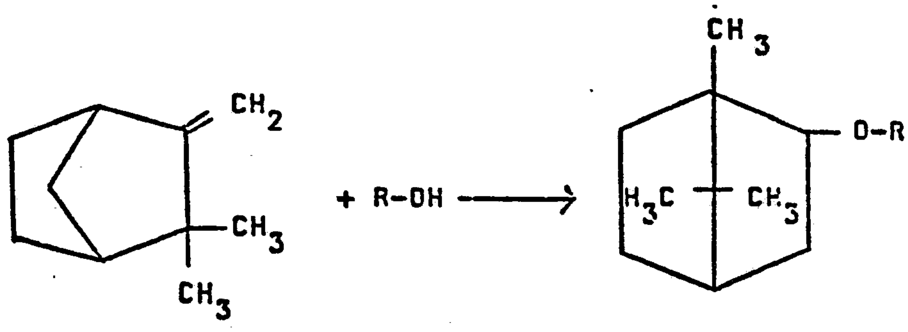

ablaufenden Reaktion findet intermediär eine Wagner-Meerwein-Umlagerung des Camphens in eine Camphenzwischenstufe statt.

Die Synthese wird bei Temperaturen zwischen Raumtemperatur (20° C) und 160°C, vorzugsweise bei 50 bis 140°C und insbosondere bei 70 bis 120°C durchgeführt. Man kann die Reaktionspartner je nach gewünschtem produkt in äquimolaren Mengen einsetzen oder auch mit einem Überschuß des einen oder anderen Partners arbeiten.

Als Katalysatoren dienen Mineralsäuren wie beispielsweise Schwefelsäure, Perchlorsäure, Phosphorsäure, Chlorsulfonsäure starke organische Säuren wie p-Toluolsulfonsäure, Methansulfonsäure, Kampfer-10-sulfonsäure, saure Ionenaustauscher oder Friedel-Crafts-Katalysatoren wie Bortrifluorid und dessen Additionsprodukte (z. B. Etherate, Eisessigkomplex), Aluminiumchlorid, Zinkchlorid und dergl. in Mengen von 0,1 bis 10, vorzugsweise 0,5 bis 6 und insbesondere 1 bis 4 Gewichts-%, bezogen auf eingesetztes Camphen.

Die Umsetzung kann in Gegenwart oder in Abwesenheit von inerten Lösemitteln vorgenommen werden. Geeignete Lösemittel sind z. B. aliphatische Kohlenwasserstoffe wie Pentan, Hexan, Benzinfraktionen, Chloroform oder Tetrachlorkohlenstoff, aromatische Kohlenwasserstoffe wia Toluol, Xylol, Chlorbenzol, cycloaliphatische Kohlenwasserstoffe wie Cyclohexan, Cyclooctan odor Ether wie Dioxan, Dibutylether oder Ethylenglykoldimethylether. Besonders empfehlenswert ist die Arbeitsweise ohne Lösemittelzugabe.

Bei der Umsetzung können im allgemeinen alle Reaktionspartner in ihrer gesamten Menge inklusive Katalysator vorgelegt werden. In manchen Fällen läuft die Reaktion leicht exotherm ab, so daß es hier vorteilhafter ist, den Katalysator und den Alkohol R-OH vorzulegen und bei der gewünschton Temperatur das Camphen zuzudosieren.

Als Beispiele für Alkohole R-OH, die mit dem Camphen umgesetzt werden, seien genannt:

Cyclopentanol, Cyclooctanol, Cyclododecanol, Cyclohexandiol-1,2, Cyclohexandiol-1,4 und 2-Methoxycyclohexanol, wobei Verbindungen mit R = a) erhalten werden.

Verbindungen mit R = b) liefern z. B. 3-Hydroxy-2,6,6-trimethyl-bicyclo-[3.1.1]-heptan (Pinanol-3), 3,7,7-Trimethyl-4-hydroxy-bicyclo-[4.1.0]-heptan (Caranol-4).

Verbindungen mit R = c) erhält man durch Umsetzen mit folgenden Alkoholen: Ethylenglykolmonocyclohexylether, Propylenglykol-mono-pinan-3-yl-ether, Neopentylglykol, Ethylenglykol, Ethylenglykol-mono-isolongifolylether.

Verbindungen, in denen R die Bedeutung von d) hat, werden z. B. aus Camphen und Cyclohexan-1,4-dimethanol, Cyclohexan-1,4-di-methanol-monomethylether oder (2,2,3-Trimethylcyclopent-7-yl)-ethanol erhalten.

Zur Herstellung von Verbindungen mit R = e) dienen z. B. folgende Alkohole: 2-Hydroxymethyl-6,6-dimethyl-bicyclo-[3.1.1]-heptan (Pinanol-10), 2-Hydroxymethyl-3,3-dimethyl-bicyclo-[2.2.1]-heptan (Camphanol), 2-(2-Hydroxyethyl)-3,3-dimethyl-bicyclo-[2.2.1]-heptan (Homocamphanol), 2-(2-Hydroxyethyl)-6,6-dimethyl-bicyclo-[3.1.1]-heptan.

Für die Herstellung von Verbindungen mit R = f) kommen schließlich in Frage: 4,8,8-Trimethyl-9-hydroxymethyl-decahydro-1,4-methano-azulen (Isolongifolol) sowie die im Handel erhältlichen Alkohole

TCD-Alkohol-M: 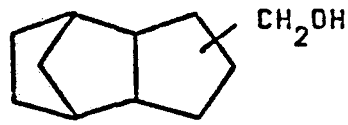 und

TCD-Alkohol-DM: 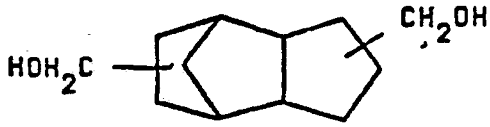

Daß die neuen Verbindungen überhaupt herstellbar sein würden und auch noch unter relativ milden Bedingungen, war keineswegs vorhersehbar, vielmehr mußte damit gerechnet werden, daß bei Einsatz der - wie oben bereits angeführt - sterisch anspruchsvollen Alkohole es entweder zu gar keiner Umsetzung kommt, oder Ausweich- oder Nebenreaktionen stattfinden. Es überrascht daher, daß die an sich bekannte, jedoch bisher nur für einige wenige niedrige Alkohole beschriebene Umsetzung bezüglich der Natur des Alkohols augenscheinlich keiner Einschränkung unterliegt.

Die Reinigung der Reaktionsprodukte erfolgt im allgemeinen nach der Entfernung des Katalysators (z. B. Wasserwäsche oder Neutralisation mittels Basen) durch Destillation, für manche Anwendungszwecke ist jedoch eine Destillation nicht erforderlich. Eine weitere Reinigungsmöglichkeit besteht in der Umkristallisation aus geeigneten Lösemitteln.

Die Reaktionsprodukte stellen niedrigviskose bis hochviskose Flüssigkeiten oder auch Festprodukte dar; sie sind farblos bis schwach gelb gefärbt.

Die neuen Verbindungen weisen z. T. ausgeprägten Riechstoffcharakter auf und sind daher für sich allein als Duftstoffe oder auch in Duftstoffkombination, also in Gemischen mit synthetischen und natürlichen Ölen, Alkoholen, Aldehyden, Ketonen, Estern usf., einsetzbar; sie eignen sich ferner zur Parfümierung von Seifen, Detergentien, Pudern, Badeölen, Haarpflegemitteln, Cremes sowie weiteren bekannten, Duftstoffe enthaltenden Zubereitungen. Ein größerer Teil der neuen Terpenether ist aufgrund seiner Konsistenz als Fixateur für Riechstoffe von Interesse; die dafür geeigneten Verbindungen haben als viskose Flüssigkeiten die Eigenschaft, die Flüchtigkeit von Riechstoffen stark herabzusetzen. Weiterhin lassen sich manche der Produkte als Lösemittel, z. B. für Harze und Lacke, verwenden.

Schließlich sind solche Vertreter der neuen Ether, die freie Hydroxylgruppen aufweisen, als reaktive Zwischenprodukte, z. B. für die Synthese von Pflanzenschutz-, Schädlingsbekämpfungs- und Arzneimitteln, geeignet.

Die folgenden Beispiele sollen die Erfindung weiter erläutern.

**Beispiel 1**

Ein mit Rühreinrichtung, Innenthermometer und Kühler ausgestatteter 1-1-Dreihalskolben wurde mit 272 g (2 Mol) Camphen, 430 g (5 Mol) Cyclopentanol und 10 g konz. Schwefelsäure beschickt. Der Kolbeninhalt wurde 24 Stunden bei 100 °C gerührt. Sodann wurde bei Zimmertemperatur zur Entfernung der Schwefelsäure mit Wasser neutralgewaschen. Nach Abdestillieren des Cyclopentanol-Überschusses erhielt man durch Fraktionierung des Rückstandes eine farblose Flüssigkeit vom Siedepunkt 85 °/0,5 mbar und einer Dichte (20 °C) von 0,923.

Ausbeute: 300 g 1,7,7-Trimathyl-bicyclo-[2.2.1]-hept-2-yl-cyclopentylether ≙ 67,6 % d. Th.
Analyse: Molgewicht = 220; ber.: 222
GC: 96 %iges Produkt
Geruch: angenehm frische, fruchtige Note

**Beispiel 2**

In der in Beispiel 1 angegebenen Apparatur wurde eine Mischung aus 272 g (2 Mol) Camphen und 600 g (3,26 Mol) Cyclododecanol in Gegenwart von 10 g konz. Schwefelsäure 24 Stunden bei 100 °C gerührt. Die Aufarbeitung wurde wie in Beispiel 1 beschrieben vorgenommen. Man erhielt eine gelbliche, viskose, geruchlose Flüssigkeit vom Siedepunkt 165 bis 168 °C/0,13 mbar, die eine Dichte (20 °C) von 0,957 besitzt.

Ausbeute: 126 g 1,7,7-Trimethyl-bicyclo-[2.2.1]-hept-2-yl-cyclododocylether ≙ 20 % d. Th.
Molgewicht: 314; ber.: 320
GC: 92 %iges Produkt

**Beispiel 3**

In der in Beispiel 1 beschriebenen Apparatur wurde 272 g (2 Mol) Camphen mit 520 g 2-Methoxycyclohexanol (4 Mol) in Gegenwart von 5 g 70 %iger Perchlorsäure 36 Stunden bei 120°C gerührt. Nach der üblichen Aufarbeitung fiel eine farblose, nahezu geruchlose Flüssigkeit vom Siedepunkt 97 bis 100 °C/0,5 mbar und einer Dichte (20 °C) von 0,935 an.

Ausbeute: 410 g 1,7,7-Trimethyl-bicyclo-[2.2.1]-hept-1-yl-2-methoxy-cyclohexylether ≙ 74,3 % d. Th.
Molgewicht: 278; ber.: 276
GC: 89 %iges Produkt

**Beispiel 4**

Unter den in Beispiel 1 angegebenen apparativen Bedingungen wurden 78 g (0,5 Mol) Camphen und 147 g (0,5 Mol) 2-(3,3-Dimethyl-bi-cyclo-[2.2.1]-hept-2-yl)-ethoxy-cyclohexanol in Gegenwart von 3 g 70 %iger Perchlorsäure 48 Stunden bei 120 °C gerührt. Nach einer Wasserwäsche und dem Abdestillieren der flüchtigen Anteile bis zu einer Innentemperatur von 150°C bei einem Vakuum bis 13 mbar erhielt man einen gelblichen, viskosen und nahezu geruchlosen Rückstand. Dichte (20 °C) 0,975.

Ausbeute: 173 g 1,7,7-Trimethyl-bicyclo-[2.2.1]-hept-2-yl-2-(3,3-dimethyl-bicyclo-[2.2.1]-hept-yl)-ethoxy-cyclohexylether = 40,2 % d. Th.
Molgewicht: 426; ber.: 430

**Beispiel 5**

Wie im Beispiel 1 beschrieben, wurden 272 g (2 Mol) Camphen mit 308 g (2 Mol) Pinanol-3 in Gegenwart von 10 g Bortrifluorid-Etherat umgesetzt. Nach der Wasserwäsche und dem Abdestillieren der leicht flüchtigen Anteile erhielt man ein farbloses, angenehm fruchtig riechendes, viskoses Destillat vom Siedepunkt 131 bis 133 °C/1 mbar und einer Dichte (20 °C) von 0,985.

Ausbeute: 145 g 1,7,7-Trimethyl-bicyclo-[2.2.1]-hept-2-yl-2,6,6-trimethyl-bicyclo-[3.1.1]-hept-3-yl-ether ≙ 50% d. Th.
Molgewicht: 292; ber.: 290
GC: 94 %iges Produkt

**Beispiel 6**

Unter den in Beispiel 1 beschriebenen apparativen Bedingungen wurden 408 g (3 Mol) Camphen und 62 g (1 Mol) Ethylenglykol in Gegenwart von 400 ml Toluol als Lösungsmittel und 5 g wasserfreiem Aluminiumchlorid 30 Stunden bei 80°C gerührt. Nach Wäsche mit Wasser und Abziehen de flüchtigen Anteile (Toluol, Camphenüberschuß und Ethylenglykol-mono-1,7,7-trimethyl-bicyclo-[2.2.1]-hept-2-yl-ether vom Siedepunkt 120 bis125 °C/13 mbar) erhielt man ein farbloses, beim Abkühlen kristallisierendes, geruchloses Destillat vom Siedepunkt 168 bis 169 °C/1,3 mbar.

Ausbeute: 223 g Ethylenglykol-di-1,7,7-trimethyl-bicyclo-[2.2.1]-hept-2-yl-ether = 67 % d. Th.
Molgewicht: 329; ber.: 334
Schmelzpunkt: 72 bis 75°C

**Beispiel 7**

In der in Beispiel 1 beschriebenen Apparatur wurden 136 g (1 Mol) Camphen und 198 g (1Mol) Ethylenglykol-mono-(3,3-dimethyl-bicyclo-[2.2.1]-hept-2-yl)-methylether in Gegenwart von 400 ml Tetrachlorkohlenstoff und 5 g Bortrifluorid-Diethyletherat 10 Stunden bei 80°C gerührt. Nach Wäsche mit Wasser und Abdestillieren der flüchtigen Anteile erhielt man bei einer Destillationstemperatur von 163 bis 167°C/1 mbar eine farblose, stark viskose, schwach nach Kampfer riechende Flüssigkeit mit einer Dichte (20°C) von 0,984.

Ausbeute: 201 g 1-(1,7,7-Trimethyl-bicyclo-[2.1.1]-hept-2-oxy)-2-(3,3-dimethyl-bicyclo-[2.2.1]-hept-2-yl)-methoxyethan = 60,2 % d. Th.
Molgewicht: 327; ber.: 334
GC: ca. 94 %iges Produkt

**Beispiel 8**

Unter den in Beispiel 1 angegebenen apparativen Bedingungen wurden 272 g (2 Mol) Camphen mit 144 g (1 Mol) 1,4-Dimethylolcyclohexan in Gegenwart von 10 g konz. Schwefelsäure 24 Stunde bei 100°C gerührt. Nach der Wasserwäsche und dem Abdestillieren der flüchtigen Bestandteile bis 200°C/20 mbar verblieb ein gelblicher, geruchloser und bei Raumtemperatur kristallisierender Rückstand.

Ausbeute: 260 g Cyclohexan-1,4-dimethyl-bis-1,7,7-trimethyl-bicyclo-[2.2.1]-hept-2-yl-ether = 62,5 % d. Th.
Schmelzpunkt: 43 bis 47°C
Molgewicht: 403; ber.: 416
Hydroxylzahl: 11

**Beispiel 9**

Unter den in Beispiel 8 angegebenen Reaktionsbedingungen wurden 272 g (2 Mol) Camphen mit 640 g (4,44 Mol) 1,4-Dimethylolcyclohexan in Gegenwart von 10 g konz. Schwefelsäure umgesetzt. Nach der Beispiel 8 entsprechenden Aufarbeitung erhielt man als Reaktionsprodukt einen gelblichen, hochviskosen, geruchlosen Rückstand.

Ausbeute: 373 g 4-Hydroxymethylcyclohexyl-1-methyl-1,7,7-trimethyl-bicyclo-[2.2.1]-hept-2-yl-ether = 64,8 % d. Th.
Molgewicht: 290; ber.: 280
Hydroxylzahl: 212; ber.: 204

**Beispiel 10**

In der in Beispiel 1 beschriebenen Apparatur wurden 272 g (2 Mol) Camphen mit 624 g (4 Mol) 2-(2,2,3-Trimethyl-cyclopent-1-yl)-ethanol in Gegenwart von 12 g ortho-Phosphorsäure 48 Stunden bei 120 °C gerührt. Nach der Wasserwäsche und dem Abdestillieren der flüchtigen Anteile erhielt man eine farblose, bei Raumtemperatur kristalline, angenehm riechende Substanz vom Siedepunkt 135 bis 138 °C/0,4 mbar.

Ausbeute: 328 g 1,7,7-Trimethyl-bicyclo-[2.2.1]-hept-2-yl-2-(2,2,3-trimethyl-cyclopent-1-yl)-ethylether = 56,2 % d. Th.
Molgewicht: 287; ber.: 292
GC: ca. 89 %iges Produkt
Smp.: 30 bis 33 °C

**Beispiel 11**

In einem 10-l-Glaskolben, ausgestattet mit Rührer, Innenthermometer, Kühler und beheizbarem Tropftrichter wurden 1664 g (16 Mol) Neopentylglykol (2,2-Dimethylpropandiol-1,3) und 112 g Bortrifluorid-Essigsäure-Komplex ($BF_3 . 2 CH_3CO_2H$) vorgelegt, worauf man auf 80°C erwärmte. Innerhalb von zwei Stunden wurden sodann 4352 g (32 Mol) geschmolzenes Camphen bei 80 bis 85°C zudosiert. Man rührte 20 Stunden bei 80 bis 85°C weiter, wusch anschließend das Reaktionsgemisch mit Wasser neutral und destillierte das nicht umgesetzte Camphen im Vakuum bis zu einer Innentemperatur von 120°C und einem Vakuum von 50 mbar ab. Als Rückstand verblieb eine gelbliche, viskose, nahezu geruchlose Flüssigkeit mit einer Dichte (20°C) von 0,945.

Ausbeute: 5118 g Rückstand
Molgewicht: 362; ber.: 376
GC: 80 % Neopentylglykol-di-1,7,7-trimethyl-bicyclo-[2.2.1]-hept-2-yl-ether 15 % Neopentylglykol-mono-1,7,7-trimethyl-bicyclo-[2.2.1]-hept-2-yl-ether

Durch Destillation über eine Kolonne konnte der Monoether (Siedepunkt: 104 °C/0,3 mbar; viskose, farblose, geruchlose Flüssigkeit) vom Diether (Siedepunkt: 165 °C/0,3 mbar, viskose, farblose, geruchlose Flüssigkeit) getrennt werden.

**Beispiel 12**

In der in Beispiel 1 beschriebenen Apparatur wurden 272 g (2 Mol) Camphen, 665 g (4 Mol) TCD-Alkohol-M (Gemisch aus 3- und 4-Hydroxy-methyl-tricyclo-[5.2.1.0$^{2,6}$]decan (Hersteller Ruhrchemie, Oberhausen-Holten) und 10 g konz. Schwefelsäure vorgelegt. Man erwärmte auf 100 °C und rührte bei dieser Temperatur 24 Stunden nach, wusch den Ansatz in Gegenwart von Ether mit Wasser neutral und destillierte nach dem

Abziehen des Ethers bis zu einer Innentemperatur von 160 °C und einem Vakuum von 13 mbar den TCD-Alkohol-Überschuß ab. Es blieb eine bräunliche, bei Raumtemperatur erstarrende, geruchlose Flüssigkeit zurück.

Ausbeute: 450 g 1,7,7-Trimethyl-bicyclo-[2.2.1]-hept-2-yl-(tricyclo-[5.2.1.$0^{2,6}$]-dec-3- und 4-yl)-methylether = 74,5 % d. Th.

Schmelzbereich: 70 bis 80 °C

Molgewicht: 312; ber.: 302

**Beispiel 13**

In der in Beispiel 1 angegebenen Apparatur wurden 272 g (2 Mol) Camphen und 546 g (3 Mol) 1-(3,3-Dimethyl-bicyclo-[2.2.1]-hept-2-yl)-propan-2-ol in Gegenwart von 12 g p-Toluolsulfonsäure 48 Stunden bei 120 °C gerührt. Nach der Wasserwäsche und dem Abdestillieren leichtflüchtiger Anteile bis zu einer Innentemperatur von 135° C/ 0,06 mbar erhielt man eine bei 123 bis 127° C bei 0,06 mbar destillierende, farblose, viskose, angenehm fruchtig riechende Flüssigkeit mit einer Dichte (20 °C) von 0,978.

Ausbeute: 144 g 1,7,7-Trimethyl-bicyclo-[2.2.1]-hept-2-yl-1-(3,3-dimethyl-bicyclo-[2.2.1]-hept-2-yl)-prop-2-yl-ether = 22,6 % d. Th.

Molgewicht: 311; ber.: 318

GC: ca. 90 %iges Produkt

**Beispiel 14**

In der in Beispiel 1 beschriebenen Apparatur wurden 136 g (1 Mol) Camphen mit 222 g (1 Mol) Isolongifolol (= 4,8,8-Trimethyl-9-hydroxymethyl-decahydro-1,4-methano-azulen) in Gegenwart von 5 g Bortrifluorid-Diethyletherat 20 Stunden bei 100°C gerührt. Nach der üblichen Aufarbeitung verblieb ein gelblicher, bei Raumtemperatur fester, nach Holz riechender Rückstand. Durch Umkristallisation erhielt man ein farbloses, holzartig riechendes Kristallisat.

Ausbeute (nach Umkrist.): 147 g 1,7,7-Trimethyl-bicyclo-[2.2.1]-hept-2-yl-isolongifolylether = 41 % d. Th.

Schmelzpunkt: 96 bis 98°C

Molgewicht: 356; ber.: 358

**Patentansprüche**

1. Terponether der Formel

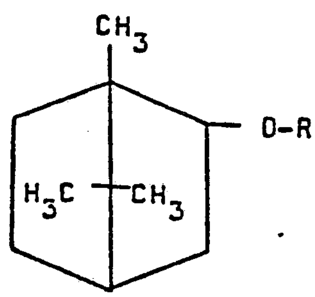

in welcher R die Bedeutung eines der nachstehend genannten Reste hat:

a) Cycloalkyl mit 5 bis 12 C-Atomen, welches durch einen Rest -O-$R^1$ substituiert sein kann, in dem $R^1$ für H, $C_1$- bis $C_{18}$-Alkyl, $C_5$- bis $C_{12}$-Cycloalkyl, $C_7$- bis $C_{12}$-Bicyclo-alkyl, oder für cycloalkyl-, bicycloalkyl- oder tricycloalkylsubstituiertes $C_1$- bis $C_6$-Alkyl steht,

b) Bicycloalkyl mit 7 bis 12 C-Atomen,

c) $C_2$- bis $C_{20}$-Alkyl, welches durch einen Rest -O-$R^2$ substituiert ist, in dem $R^2$ die Bedeutung von $C_5$- bis $C_{12}$-Cycloalkyl, von $C_7$- bis $C_{12}$-Bicycloalkyl oder von cycloalkyl-, bicycloalkyl- oder tricycloalkylsubstituiertem $C_1$- bis $C_6$-Alkyl hat,

d) $C_1$- bis $C_4$-Alkyl, das durch $C_5$- bis $C_{12}$-Cycloalkyl substituiert ist, wobei der Cycloalkylrest einen Substituenten -$CH_2$-O-$R^1$ mit $R^1$ wie unter a) angegebenen tragen kann,

e) $C_1$- bis $C_4$-Alkyl, das durch $C_7$- bis $C_{12}$-Bicycloalkyl substituiert ist,

f) $C_1$- bis $C_4$-Alkyl, das durch $C_8$- bis $C_{11}$-Tricycloalkyl substituiert ist, wobei der Tricycloalkylrest seinerseits durch -$CH_2O$-$R^1$ mit $R^1$ wie unter a angegeben substituiert sein kann.

2. Verfahren zur Herstellung der Terpenether nach Anspruch 1 durch Umsetzen von Camphen mit einem Alkohol bei 20 bis 140°C in Gegenwart eines sauren Katalysators und gewünschtenfalls eines inarten Lösungsmittels,

dadurch gekennzeichnet, daß als Alkohol eine Verbindung der Formel R-OH mit R wie in Anspruch 1 angegeben eingesetzt wird.

## Claims

1. A terpene ether of the formula

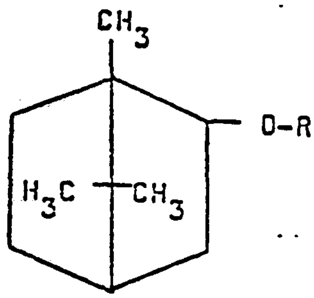

in which R denotes one of the radicals mentioned below:

a) cycloalkyl which has 5 to 12 carbon atoms and which can be substituted by a radical $-O-R^1$, in which $R^1$ represents H, $C_1$- to $C_{18}$-alkyl, $C_5$- to $C_{12}$-cycloalkyl, $C_7$- to $C_{12}$-bicycloalkyl, or $C_1$- to $C_6$-alkyl which is substituted by cycloalkyl, bicycloalkyl or tricycloalkyl,

b) bicycloalkyl with 7 to 12 carbon atoms,

c) $C_2$- to $C_{20}$-alkyl, which is substituted by a radical $-O-R^2$, in which $R^2$ denotes $C_5$- to $C_{12}$-cycloalkyl, $C_7$- to $C_{12}$-bicycloalkyl, or $C_1$- to $C_6$-alkyl which is substituted by cycloalkyl, bicycloalkyl or tricycloalkyl,

d) $C_1$- to $C_4$-alkyl which is substituted by $C_5$- to $C_{12}$-cycloalkyl, it being possible for the cycloalkyl radical to carry a substituent $-CH_2-O-R^1$, where $R^1$ is as described under a),

e) $C_1$- to $C_4$-alkyl, which is substituted by $C_7$- to $C_{12}$-bicycloalkyl, or

f) $C_1$- to $C_4$-alkyl, which is substituted by $C_8$- to $C_{11}$-tricycloalkyl, it being possible for the tricycloalkyl radical to be in turn substituted by $CH_2-O-R^1$, where $R^1$ is as described under a).

2. A process for the preparation of a terpene ether as claimed in claim 1 by reaction of camphene with an alcohol at 20 to 140°C in the presence of an acid catalyst and, if desired, of an inert solvent, which comprises using a compound of the formula R-OH, where R is as defined in claim 1, as the alcohol.

## Revendications

1. Ethers terpéniques répondant à la formule suivante

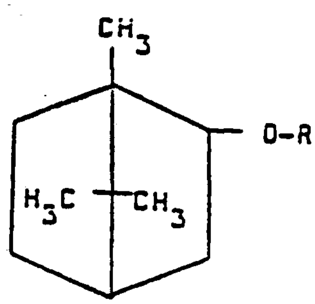

dans laquelle R représente l'un des radicaux mentionnés ci-dessous:

a) un radical cycloalkyle contenant de 5 à 12 atomes de carbone, éventuellement porteur d'un radical $-O-R^1$ dans lequel $R^1$ représente H, un alkyle en $C_1$-$C_{18}$, un cycloalkyle en $C_5$-$C_{12}$, un bicycloalkyle en $C_7$-$C_{12}$ ou un alkyle en $C_1$-$C_6$ porteur d'un cycloalkyle, d'un bicycloalkyle ou d'un tricycloalkyle,

b) un radical bicycloalkyle contenant de 7 à 12 atomes de carbone,

c) un radical alkyle en $C_2$-$C_{20}$ qui porte un radical -O-$R^2$ dans lequel $R^2$ représente un cycloalkyle en $C_5$-$C_{12}$, un bicycloalkyle en $C_7$-$C_{12}$ ou un alkyle en $C_1$-$C_6$ porteur d'un cycloalkyle, d'un bicycloalkyle ou d'un tricycloalkyle,

d) un radical alkyle en $C_1$-$C_4$ qui porte un radical cycloalkyle en $C_5$ à $C_{12}$, ce radical cycloalkyle pouvant porter un substituant -$CH_2$-O-$R^1$ dans lequel $R^1$ a la signification donnée sous a),

e) un radical alkyle en $C_1$-$C_4$ qui porte un bicycloalkyle en $C_7$-$C_{12}$,

f) un radical alkyle en $C_1$-$C_4$ qui porte un radical tricycloalkyle en $C_8$-$C_{11}$, ce radical tricycloalkyle pouvant de son côté porter un substituant -$CH_2$-O-$R^1$ dans lequel $R^1$ a la signification donnée sous a).

2. Procédé de préparation des éthers terpéniques selon la revendication 1 par réaction du camphène avec un alcool, à une température de 20 à 140°C, en présence d'un catalyseur acide et, si on le désire, d'un solvant inerte, procédé caractérisé en ce qu'on utilise comme alcool un composé répondant à la formule R-OH dans laquelle R a la signification donnée à la revendication 1.